# EUROPÄISCHE PATENTANMELDUNG

(11) **EP 1 815 814 A2**
(43) Veröffentlichungstag der Anmeldung: **08.08.2007**
(21) Anmeldenummer: 07002218.1
(22) Anmeldetag: 01.02.2007
(51) Int. Cl.: A61B 19/00, A61B 6/00

(54) **Verfahren und Vorrichtung zur Koordinatentransformation bei navigationsgeführten Eingriffen**

(30) Priorität: 02.02.2006 DE 102006004793
(71) Anmelder: Ziehm Imaging GmbH, 90451 Nürnberg (DE)
(72) Erfinder: Hörndler, Klaus, 90482 Nürnberg (DE); Fleischmann, Christof, 91096 Möhrendorf (DE)

(57) **Zusammenfassung**

Die Erfindung betrifft ein Verfahren und eine Vorrichtung zur Koordinatentransformation bei navigationsgeführten Eingriffen unter Verwendung einer bildgebenden Diagnostikeinrichtung, insbesondere einer Röntgendiagnostikeinrichtung, und eines Lageerfassungssystems. Hierzu wird eine mehrfach reproduzierbar verstellbare Diagnostikeinrichtung zu einem Untersuchungsobjekt plaziert und im Falle des Vorhandenseins eines Fahrstativs dieses bezüglich des Fußbodens lösbar fixiert. Die Diagnostikeinrichtung weist in der Nähe des Bildempfängers eine räumliche Referenzstruktur auf, die mit einem Zeiger eines Lageerfassungssystems antastbar ist. Aus der bekannten Geometrie der räumlichen Referenzstruktur und den mit den vom Lageerfassungssystem ermittelten Koordinaten der Antastpunkte ist eine Koordinatentransformation zwischen dem Koordinatensystem der Diagnostikeinrichtung und dem Koordinatensystem des Lageerfassungssystems hergestellt. Ein von dem Lageerfassungssystem erfaßtes Instrument kann nach der Registrierungsprozedur in rekonstruierten Bildern der Diagnostikeinrichtung navigiert werden.

## Beschreibung

Die Erfindung betrifft ein Verfahren und eine Vorrichtung zur Koordinatentransformation bei navigationsgeführten Eingriffen unter Verwendung einer bildgebenden Diagnostikeinrichtung und eines Lageerfassungssystems.

Medizinische Eingriffe an lebenden Objekten werden zunehmend mittels Navigationsunterstützung durchgeführt. Darunter versteht man die mittels eines Lageerfassungssystems unterstützte Führung eines Instrumentes relativ zu einem in Behandlung stehenden Gewebebereichs des Objektes. Von besonderem Interesse ist die Navigation in Bereichen, die sich einer optischen Kontrolle des Operateurs entziehen, weil das Instrument beispielsweise in das Innere des Objektes eingeführt wurde. Hierzu wird die Führung des Instrumentes, beispielsweise eines Katheders, in einem virtuellen 3D-Volumen vorgenommen, welches mittels eines bildgebenden Verfahrens vor oder während der Operation erzeugt wurde. Eine häufige Anwendung ist, mit Hilfe einer Röntgendiagnostikeinrichtung eine Reihe von 2D-Projektionsaufnahmen bekannter Projektionsgeometrie zu erzeugen und aus diesen 2D-Aufnahmen einen 3D-Volumendatensatz zu erzeugen. Der Volumendatensatz wird an ein Navigationssystem übergeben, das über ein Lageerfassungssystem für von diesem erfaßbaren Marken verfügt. Um eine Navigation mit hoher Genauigkeit möglich zu machen, wird das Koordinatensystem des Lageerfassungssystem mit dem Koordinatensystem des 3D-Volumendatensatzes abgeglichen. Dieser Vorgang wird üblicherweise "Registrierung" genannt. Bei der Registrierung wird beispielsweise ein Phantom, das röntgenpositive Marken und vom Lageerfassungssystem erfaßbare Marken in einer festen Raumbeziehung zueinander enthält, verwendet.

Aus der DE 102 02 091 A1 ist eine Vorrichtung und ein Verfahren zur Ermittlung einer Koordinatentransformation unter Verwendung eines Phantoms bekannt, an dem röntgenpositive Marken und von einem Lageerfassungssystem erfaßbare Marken in einer festen räumlichen Beziehung zueinander angeordnet sind. Bei einem Scan zur Erzeugung einer Reihe von 2D-Röntgenprojektionsaufnahmen werden die Koordinaten der röntgenpositiven Marken im rekonstruierten 3D-Volumen ermittelt und zum Abgleich an das Lageerfassungs- und Navigationssystem übermittelt.

Aus der deutschen Offenlegungsschrift DE10360025A1 ist ein Verfahren zur Koordinatentransformation bekannt, bei dem von einem Lageerfassungssystem erfaßbare Marken an einer Bildaufnahmeeinrichtung, insbesondere einem Röntgenstrahlenempfänger angeordnet sind. Dieses Verfahren weist den Nachteil auf, daß bei Einhüllen der Bildaufnahmeeinrichtung mit einer Sterilabdeckfolie zwischen die Marken und dem Lageerfassungssystem ein den Lageerfassungsvorgang störendes Medium, nämlich die Sterilabdeckfolie eingebracht wird.
Des weiteren sind in der Regel eine Reihe von Marken in Folge der Ausrichtung der Vorrichtung bezüglich der Patientenliege und des Lageerfassungssystems nicht erfaßbar, wodurch die Genauigkeit eines Referenzierungsvorganges begrenzt ist. Zudem ist die Röntgendiagnostikeinrichtung durch die an dem Röntgenstrahlenempfänger angeordneten Marken nicht ohne mechanischen Umbau mit einem anderen Typus von Lageerkennungssystem betreibbar.

Aus der deutschen Patentschrift DE19917867B4 ist ein Verfahren und eine Vorrichtung zur Koordinatentransformation für eine Röntgendiagnostikeinrichtung bekannt, wobei vorgesehen ist, an dem Röntgenstrahlenempfänger eine von einem Lageerfassungssystem erfassbare Referenzstruktur lösbar anzubringen. Es ist vorgesehen, nach erfolgter Registrierung die Referenzstruktur vom Röntgenstrahlenempfänger abzunehmen. Dieses Verfahren weist den Nachteil auf, daß die Masseverteilung der Röntgendiagnostikeinrichtung im Bereich eines verstellbaren C-Bogens bei der Registrierung und bei der diagnostischen Bildaufnahme unterschiedlich ist und die Kinematik der Röntgendiagnostikeinrichtung im Hinblick auf den Einfluß der Zusatzmasse auf die Verwindung des C-Bogens berücksichtigt werden muß.

Aufgabe der Erfindung ist es, bei navigationsgeführten Eingriffen unter Verwendung einer mehrfach verstellbaren bildgebenden Diagnostikeinrichtung und eines beliebigen Lageerfassungssystems hinreichender Genauigkeit die Koordinatentransformation zwischen dem Koordinatensystem des Lageerfassungssystem und dem Koordinatensystem der bildgebenden Diagnostikeinrichtung, in dem die Kinematik der für die Bilderzeugung erforderlichen Mittel beschrieben wird, intraoperativ auf einfache und kostengünstige Weise mit hoher Genauigkeit zu ermitteln, wobei unter den Begriffen "Bilderzeugung" und "Bildgebung" auch solche Verfahren verstanden werden sollen, die es erlauben, aus einer Reihe von Meßwerten ein Bild zu rekonstruieren.

Die Aufgabe der Erfindung wird dadurch gelöst, daß an wenigstens einer Stelle der mehrfach verstellbaren bildgebenden Diagnostikeinrichtung eine räumliche Referenzstruktur angeordnet ist und diese mittels eines von einem beliebigen Lageerfassungssystems hinreichender Genauigkeit erfaßbaren Zeigers antastbar ist. Die Koordinaten der Zeigerspitze werden beim Antasten von dem Lageerfassungssystem ermittelt und in einem Speicher zwischengespeichert. Aus der bekannten Geometrie der räumlichen Referenzstruktur bezüglich eines vom Hersteller der Diagnostikeinrichtung gewählten und bezüglich diesem ortsfesten Koordinatensystems sowie aus den ermittelten Koordinaten der Antastpunkte wird von einer Recheneinheit eine Transformationsmatrix errechnet, mittels der die Koordinatentransformation zwischen dem Koordinatensystem des Lageerfassungssystems und dem der Diagnostikeinrichtung rekursiv vorgenommen werden kann. Die Bestimmung der Transformationsmatrix erfolgt nach bekannten Rechenverfahren, wobei wahlweise rekursive und iterative Verfahren angewandt werden.

Die Erfindung wird an Hand der Abbildungen näher erläutert.

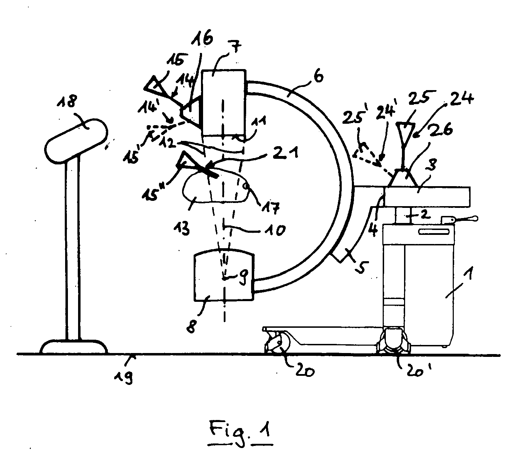

In Fig. 1 ist als Beispiel für eine bildgebende Diagnostikeinrichtung eine mobile Röntgendiagnostikeinrichtung mit einem auf Rollen 20, 20' längs des Fußbodens 19 verschiebbaren Gerätewagen 1, der einen mehrfach verstellbaren C-Bogen 6 trägt, dargestellt. Die Mittel zur Bilderzeugung der bildgebenden Diagnostikeinrichtung sind im Fall des gewählten Beispiels eine Röntgenstrahlenquelle 8 und ein Röntgenstrahlenempfänger 7, die an den Enden eines C-Bogens 6, der längs seines Umfanges in einer C-Bogenhalterung 5 um den Mittelpunkt 17 verschieblich gelagert ist, angeordnet sind. Die C-Bogenhalterung 5 ist an dem Gerätewagen 1 mehrfach verstellbar gelagert. Die C-Bogenhalterung 5 ist mit einem Schwenklager 4 an einer Horizontalführung 3 um eine horizontale Achse schwenkbar gelagert. Die Horizontalführung 3 ist an einer Säule 2 höhenverstellbar und um die senkrechte Achse der Säule 2 drehbar gelagert. Es sind vorzugsweise alle Verstelleinrichtungen des C-Bogens 6 mit Positionsmeßsensoren ausgerüstet, deren Meßwerte einer zentralen Bewegungssteuerung der Röntgendiagnostikeinrichtung zugeführt werden. Es ist vorgesehen, alle Verstellachsen wahlweise einzeln oder in der Gesamtheit durch Bremsen arretierbar auszuführen. Insbesondere ist es vorgesehen, die Rollen 20, 20' mit einer Feststellbremse auszurüsten. Vorzugsweise sind die Verstellbewegung des C-Bogens in der Halterung (Orbitalbewegung), die Verstellung in der Horizontalführung 3 und die vertikale Verstellung in der Säule elektromotorisch verstellbar ausgeführt, wobei die in den Verstellachsen angeordneten Motoren von einer zentralen Bewegungssteuerung der Röntgendiagnostikeinrichtung gesteuert werden. Entscheidend ist, daß die Lage aller Verstellachsen, die zur Gewinnung von diagnostischen Bildern oder Bilddatensätzen während einer Untersuchung verstellt werden müssen, durch jeweils geeignete Positionssensoren erfaßt werden und daß wenigstens die Verstellachsen, die keine Positionssensoren aufweisen, während der Untersuchung in einer festgelegten Position reproduzierbar arretiert werden. Unter derartigen Bedingungen ist die Kinematik der Röntgenstrahlenquelle 8 und des Röntgenstrahlenempfängers 7 als Funktion der Wertetupel der Meßwerte der Positionssensoren vorhersagbar. Dazu wird die Kinematik der Röntgendiagnostikeinrichtung in der Regel vor Auslieferung an den Kunden mit geeigneten Mitteln vermessen, um die für jedes Gerät individuelle aber reproduzierbare Verwindung des C-Bogens zu berücksichtigen. In der Praxis werden die Abweichungen der ermittelten Kinematik von der Kinematik eines sich nicht verwindenden, kreisbogenförmigen C-Bogens in Look-up-Tabellen im Steuer- oder Bildverarbeitungsrechner der Röntgendiagnostikeinrichtung abgespeichert und bei der Rekonstruktion der Röntgenprojektionsgeometrie heran gezogen.

In Fig. 1 ist eine Referenzstruktur 16 schematisch dargestellt, die im Ausführungsbeispiel am Röntgenstrahlenempfänger 7 angeordnet ist. Im Beispiel der Fig. 1 handelt es sich bei der Referenzstruktur 16 um einen Pyramidenstumpf. Mittels eines Zeigers 14, 14' werden Punkte oder Flächen der Referenzstruktur 16 angetastet, wobei die Lage der Spitze des Zeigers 14, 14' über die Ermittlung der Lage der Markenanordnung 15, 15' im Raum durch das Lageerfassungssystem erfolgt, und die Kenntnis der Geometrie des als starren Körpers angenommenen Zeigers 14 in die Lageberechnung eingeht.

Das Antasten des Zeigers 14, 14' an der Referenzstruktur 16 kann durch eine Bedienperson durch punktuelles Antasten erfolgen, wobei durch geeignete Maßnahmen sicher gestellt wird, daß der Röntgenstrahlenempfänger 7 und der C-Bogen 6 nicht durch eine zu starken Andruckkraft des Zeigers auf die Referenzstruktur elastisch oder bleibend verlagert wird. Diese Maßnahmen können beispielsweise darin bestehen, daß ein Kraftbegrenzer in der Referenzstruktur 16 oder in dem Zeiger 14, 14' vorgesehen ist. Es ist ferner vorgesehen, die Mittel zur Bilderzeugung der Diagnostikeinrichtung vor dem Antasten der Referenzstruktur 16 mit dem Zeiger 14 in eine festgelegte Parkposition zu verlagern, in der die Mittel zur Bilderfassung beim Antasten mit dem Zeiger 14 weitgehend unempfindlich gegenüber der Antastkraft sind. Im Beispiel der Röntgendiagnostikeinrichtung aus Fig. 1 mit einer an dem Röntgenstrahlenempfänger 7 angeordneten Referenzstruktur 16 wird als Parkposition vorzugsweise diejenige Einstellung gewählt, bei der der Röntgenstrahlenempfänger 7 am Ende des Verstellbereiches der C-Bogenhalterung 5 in unmittelbarer Nähe zu der Horizontalführung 3 zu liegen kommt.

In Fig. 1 ist ferner eine weitere Referenzstruktur 26 gezeigt, die im Beispiel der Fig. 1 an der Horizontalführung 3 angeordnet ist und mit einem Zeiger 24, 24' mit Markenanordnung 25, 25' antastbar ist. Die bildgebende Diagnostikeinrichtung kann wahlweise mit einer einzigen der beiden Referenzstrukturen 16, 26 ausgestattet sein; es ist jedoch im Rahmen der Erfindung vorgesehen, an der bildgebenden Diagnostikeinrichtung zwei Referenzstrukturen 16, 26 und bei Bedarf weitere nicht dargestellte Referenzstrukturen vorzusehen, wobei die einzelnen Referenzstrukturen in ihrer räumlichen Ausprägung derart unterscheiden, daß sie durch den Vorgang des Antastens automatisch erkennbar sind.

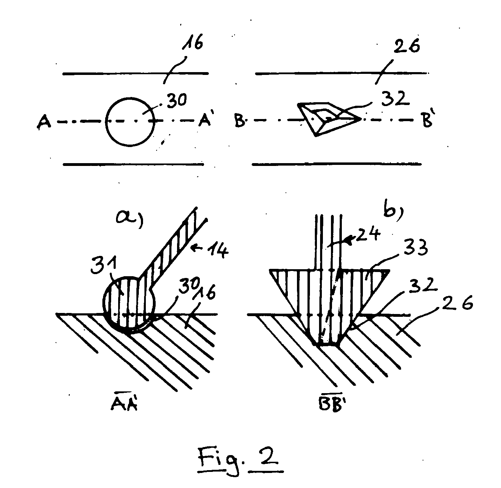

In Fig. 2 sind Einzelheiten einer erfindungsgemäßen Referenzstruktur 26 dargestellt. In der Oberfläche der Referenzstruktur 26 ist eine kalottenförmige Vertiefung 30 eingelassen. Diese Vertiefung 30 nimmt eine Kugel einer Kugelspitze 31 eines Zeigers 14 paßgenau auf; die Kugel der Kugelspitze 31 entspricht der eingeschriebenen Kugel der Kalotte 30. In Fig. 2a ist die erfindungsgemäße kalottenförmige Vertiefung 30 in der Draufsicht und im Schnitt dargestellt. Eine kalottenförmige Vertiefung 31 ist als Antastpunkt für eine Kugelspitze 31 eines Zeigers 14 auch im sterilen Bereich einer bildgebenden Diagnostikeinrichtung verwendbar. Es ist vorgesehen, daß eine 20-30 Mikrometer dicke Sterilabdeckungsfolie zwischen der Kugelspitze 31 und der kalottenförmigen Vertiefung 30 eingeschlossen werden kann. Bei der Koordinatentransformation kann die Dicke der Folie berücksichtigt werden. Wird der Zeiger 14 mit der Kugelspitze 31 von einer Bedienperson wie in Fig. 2a dargestellt, in eine kalottenförmige Vertiefung 30 eingebracht und wird der Zeiger von der Bedienperson derart bewegt, daß die Längsachse des Zeigers 14 eine Kegelmantelfläche oder einen Abschnitt davon beschreibt und ermittelt das Lageerfassungssystem 18 während der Bewegung des Zeigers mehrfach die Position des Mittelpunktes der Kugelspitze 31, so kann in dem Wissen, daß sich die Kugelspitze 31 stets an der gleichen Stelle im Raum befunden hat, die Position der Kugelspitze 31 mit wesentlich höherer Genauigkeit ermittelt werden, als dies mit einer einzigen Messung möglich wäre.

In Fig. 2b ist eine pyramidenstumpfförmige Vertiefung 32 in einer Referenzstruktur 26 in der Draufsicht und im Schnitt dargestellt. Eine paßgenau in diese pyramidenstumpfförmige Vertiefung 32 passende Pyramidenstumpfspitze 33 an einem Zeiger 24 ist in der Vertiefung lösbar angeordnet. Die Erfindung sieht vor, daß die Pyramidenstumpfspitze 33 während der Positionsbestimmung durch das Lageerfassungssystem 18 nicht von Hand gehalten wird sondern beispielsweise durch Federkraft, Magnetkraft oder durch eine nicht dargestellte mechanische Rastvorrichtung an der Referenzstruktur 26 gehalten wird. An der bildgebenden Diagnostikeinrichtung sind erfindungsgemäß mehrere pyramidenstumpfförmige Vertiefungen 32 vorgesehen, in die nacheinander die Pyramidenstumpfspitze 33 des Zeigers 24 eingeschoben wird. Im Beispiel der Fig. 2b ist als Zeigerspitze eine Pyramidenstumpfspitze 32 dargestellt; im Rahmen der Erfindung ist die Form der Spitze jedoch in weiten Grenzen veränderbar. Wesentlich ist hierbei nur, daß eine eindeutige Orientierung des Zeigers sicher gestellt ist; beispielsweise darf der Zeiger an der Spitze keine Symmetrie bezüglich der Zeigerachse aufweisen. Bedingt durch eine vorzugsweise kantige Form oder eine Form mit kleinen Krümmungsradien der Vertiefung ist es in der Praxis nicht möglich, eine derartige Vertiefung im sterilen Bereich vorzusehen, Eine Sterilabdeckfolie würde entweder beim Einschieben der Pyramidenstumpfspitze 32 in die pyramidenstumpfförmige Vertiefung 31 angeschnitten und dadurch unbrauchbar gemacht werden oder die Spitze könnte - beispielsweise in Folge von Faltenbildung der Folie - nicht in die Vertiefung eingeschoben werden können. Eine derartige Referenzstruktur ist daher vorzugsweise im nicht-sterilen Bereich der bildgebenden Diagnostikeinrichtung, beispielsweise an der Oberseite der Horizontalführung 3, vorzusehen.

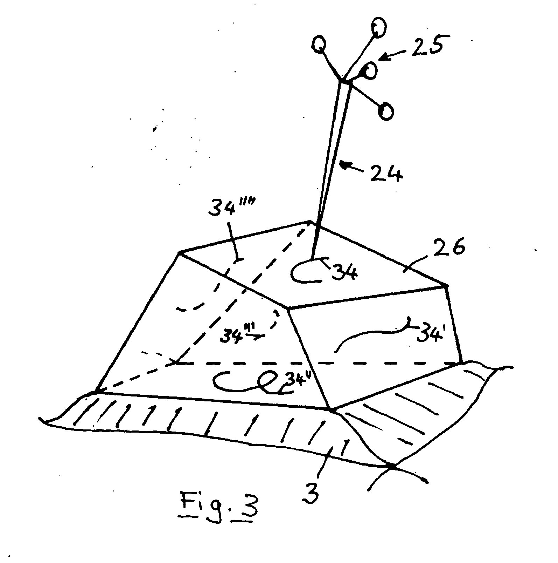

In Fig. 3 ist beispielhaft eine aus Bahnkurvenabschnitten 34, ... 34"" gebildete Bahnkurve der Spitze eines Zeigers 24 mit einer Markenanordnung 25 gezeigt. Die Bahnkurvenabschnitte 34, ... 34"" kommen dadurch zustande, daß der Zeiger 24 mit seiner Spitze von einer Bedienperson nacheinander auf den Flächen einer im Beispiel der Fig. 3 als schiefen Pyramidenstumpf dargestellten Referenzstruktur 26 in Kontakt mit dieser bewegt wird. Das Lageerfassungssystem 18 erfaßt die Bewegung der Spitze des Zeigers 24 im Raum mit einer vorgegebenen Meßtaktrate und übergibt die Ortsvektoren der Bahnkurvenabschnitte 34, ... 34"" in einem geeigneten Format, beispielsweise als Punktreihe oder als Vektorgrafik, an einen Auwerterechner der Diagnostikeinrichtung, in der die Summe der experimentell ermittelten Bahnkurvenabschnitte 34, ...34"" mittels eines "best fit" an die bekannte Oberflächengeometrie der Referenzstruktur 26 angepaßt wird. Hierdurch wird die Lage der Referenzstruktur 26 sehr genau bezüglich des Koordinatensystems des Lageerfassungssystems 18 ermittelt, wodurch die Genauigkeit der Koordinatentransformation wegen Überbestimmung des Gleichungssystems mit hoher Genauigkeit ermittelbar ist. Wird beispielsweise ein singulärer Punkt auf der Referenzstruktur 26 mit dem Zeiger 24 angefahren und angetastet, so ist aus der Lage der Marken 25 durch Addition eines dem Zeiger 24 eigenen Vektors die Lage des Antastpunktes errechenbar. Verharrt die Zeigerspitze auf dem singulären Antastpunkt und wird der Schaft des Zeigers 24 mit den Marken 25 im Raum verschwenkt, so kann nach der Methode der kleinsten Fehlerquadrate die Lage des Antastpunktes mit höherer Genauigkeit bestimmt werden, als dies bei einem einzigen Antasten möglich wäre.

Bei den Antastvorgängen muß sicher gestellt sein, daß die bildgebende Diagnostikeinrichtung durch die Antastkraft nicht elastisch verformt oder bleibend verlagert wird. Es ist im Rahmen der Erfindung vorgesehen, eine Überwachung der Antastkraft vorzunehmen, beispielsweise mittels eines piezoelektrischen Sensors in der Zeigerspitze. Damit können beispielsweise Antastvorgänge mit unzulässig hoher Antastkraft von der Berechnung der Koordinatentransformation ausgeschlossen werden. Es ist ferner vorgesehen, den Antastzeitpunkt der Zeigerspitze auf der Referenzstruktur bei punktförmigem Antasten elektromagnetisch oder optisch zu bestimmen. Beispielsweise würde das Lageerfassungssystem 18 lediglich im Antastzeitpunkt die Koordinaten der im Zeiger integrierten Markeranordnung bestimmen.

Im Beispiel der auf den Pyramidenstumpfflächen verlaufenden Bahnkurvenabschnitten 34, ... 34"" werden diese nach bekannten mathematischen Methoden der Fehlerrechnung an die pyramidenstumpfförmige Referenzstruktur 26 angepaßt. Um eine Koordinatentransformation unter Verwendung einer pyramidenstumpfförmigen Referenzstruktur 26 durchführen zu können, muß der Pyramidenstumpf derart gestaltet sein, daß er bei einer Drehung um eine beliebige Achse allenfalls bei einer Drehung um 360 Grad in sich selbst übergeht. Diese Bedingung ist beispielsweise durch einen schiefen Pyramidenstumpf, wie er in Fig. 3 schematisch dargestellt ist, erfüllt.

Für den Fall, daß die Koordinatentransformation aus der Lagebestimmung einzelner Antastpunkte errechnet werden soll, sind wenigstens vier nicht in einer Ebene liegende Meßpunkte an einer Referenzstruktur 26 anzutasten. Alternativ können auch drei eine Ebene definierende Meßpunkte, die kein gleichschenkliges Dreieck bilden, angetastet werden und die Bestimmung des Koordinatensystems der Diagnostikeinrichtung durch Anwendung einer festzulegende Konvention errechnet werden. Wählt man beispielsweise die drei Meßpunkte auf der Referenzstruktur 26 derart, daß sie ein ungleichschenkliges, rechtwinkliges Dreieck bilden, so könnte eine Konvention beispielsweise lauten: Der Eckpunkt des Dreiecks mit dem rechten Winkel wird als Koordinatenursprung gewählt, die längere Kathede des Dreiecks gibt - vom Koordinatenursprung aus gesehen - die Richtung der x-Achse vor, die kürzere Kathede des Dreiecks gibt - vom Koordinatenursprung aus gesehen - die Richtung der y-Achse vor und die z-Achse zeigt in Richtung des Vektorproduktes aus dem Vektor der x-Achse und dem Vektor der y-Achse. Die Bedingung, daß die drei Meßpunkte ein rechtwinkliges Dreieck aufspannen, ist keineswegs erforderlich. So kann beispielsweise der von der längsten und der zweitlängsten Seite des Dreiecks eingeschlossene Eckpunkt als Koordinatenursprung festgelegt werden, die längste Seite als x-Achse und die z-Achse in Richtung des Vektorproduktes aus den Seitenvektoren der längsten und der zweitlängsten Seite festgelegt werden. Die y-Achse liegt in der von den drei Punktes des Dreiecks aufgespannten Ebene und steht senkrecht auf der x-Achse.

Das Lageerfassungssystem 18 kann ein optisches (Infrarotsystem), elektromagnetisches, oder ein auf der Vermessung eines Magnetfeldes oder des Schwerefeldes der Erde beruhendes System sein.

Ein von dem Lageerfassungssystem 18 erfaßtes Instrument 21 in Fig. 1 mit Marken 15" kann nach der Registrierungsprozedur beispielsweise in einem rekonstruierten 2D- oder 3D-Röntgenmodell, einem CT-Volumen, einem Ultraschall-Volumen oder einer Magnetresonanzvolumen navigiert werden. Das heißt, daß die Bewegungsabläufe des Instrumentes 21 an Hand eines schematisch dargestellten Modells des Instrumentes 21 in einem rekonstruierten 2D- oder 3D-Modell der Patientenrealität für einen Operateur auf einem Monitor verfolgbar sind. Dies ist insbesondere dann von Vorteil, wenn der Arbeitsbereich des Instrumentes 21, beispielsweise die Spitze eines Bohrers, für den Operateur im sichtbaren Bereich des elektromagnetischen Spektrums nicht erkennbar ist.

### Verzeichnis der Abbildungen:

- Fig. 1:: Bildgebende medizinische Einrichtung mit Referenzstrukturen und Lageerfassungssystem
- Fig. 2:: Verschiedenartige Vertiefungen auf Referenzstrukturen
- Fig. 3:: Bahnkurvenabschnitte einer Zeigerspitze auf einer Referenzstruktur

### Bezugszeichenliste:

- 1: Gerätewagen
- 2: Säule
- 3: Horizontalführung
- 4: Schwenklager
- 5: C-Bogenhalterung
- 6: C-Bogen
- 7: Röntgenstrahlenempfänger
- 8: Röntgenstrahlenquelle
- 9: Brennfleck
- 10: Zentralstrahl
- 11: Eingangsfenster
- 12: Strahlenkegel
- 13: Untersuchungsobjekt
- 14,14': Zeiger
- 15,15', 15": Markenanordnung
- 16: Referenzstruktur
- 17: Mittelpunkt des C-Bogens
- 18: Lageerfassungssystem
- 19: Fußboden
- 20, 20': Rolle
- 24, 24': Zeiger
- 25, 25': Markenanordnung
- 26: Referenzstruktur
- 30: kalottenförmige Vertiefung
- 31: Kugelspitze
- 32: pyramidenstumpfförmige Vertiefung
- 33: Pyramidenstumpfspitze
- 34,... 34"": Bahnkurvenabschnitt

## Patentansprüche

1. Verfahren zur Ermittlung der Koordinatentransformation bei navigationsgeführten Eingriffen zwischen einer bildgebenden Diagnostikeinrichtung mit wenigstens einer Referenzstruktur 16, 26, die eine bekannte Oberflächengeometrie und Orientierung gegenüber der bildgebenden Diagnostikeinrichtung aufweist, und einem Lageerfassungssystem 18,
**dadurch gekennzeichnet,**
- **daß** die Referenzstruktur 16, 26 mit der Spitze eines Zeigers 14, 24, der von dem Lageerfassungssystem 18 erfaßbare Marken 15, 25 aufweist, an wenigstens drei ein nichtgleichschenkliges Dreieck bildenden Antastpunkten durch Antasten abgetastet wird,
- **daß** die Koordinaten der Antastpunkte mittels des Lageerfassungssystems 18 in einem Koordinatensystem desselben ermittelt und an einen Auswerterechner der bildgebenden Diagnostikeinrichtung übermittelt werden und
- **daß** aus den Koordinaten der Antastpunkte im Koordinatensystem des Lageerfassungssystems 18 und aus der bekannten Oberflächengeometrie der Referenzstruktur 16, 26 in einem Koordinatensystem der bildgebenden Diagnostikeinrichtung mittels eines Rechenprogrammes in dem Auswerterechner eine Transformationsmatrix errechnet wird, die die Koordinatensysteme des Lageerfassungssystems 18 und der bildgebenden Diagnostikeinrichtung rekursiv ineinander überführt.

2. Verfahren nach Anspruch 1,
**dadurch gekennzeichnet, daß** die Referenzstruktur 16 an den Orten der Antastpunkte kalottenförmige Vertiefungen 30 aufweist, welche eine Kugelspitze 31 des Zeigers 14 passgenau aufnimmt und daß beim Antasten eines Antastpunktes die Kugelspitze 31 in der jeweils angetasteten kalottenförmigen Vertiefung 30 derart verschwenkt wird, daß die Längsachse des Zeigers 14 einem Kegelmantelabschnitt beschreibt und daß während der Verschwenkung des Zeigers 14 die Koordinaten der Kugelspitze 31 des Zeigers 14 mittels des Lageerfassungssystems 18 in dessen Koordinatensystem erfaßt werden.

3. Verfahren nach Anspruch 1,
**dadurch gekennzeichnet, daß** die Referenzstruktur 26 an den Orten der Antastpunkte Vertiefungen aufweist, welche eine anisotrope Spitze des Zeigers 24 ausschließlich in einer eindeutigen Orientierung passgenau aufnimmt und daß beim Antasten eines Antastpunktes die Spitze des Zeigers 24 passgenau in die jeweilige Vertiefung eingeführt wird und im Anschluß daran die Orientierung der Spitze des Zeigers 24 mittels des Lageerfassungssystems 18 in dessen Koordinatensystem erfaßt wird.

4. Verfahren nach Anspruch 1,
**dadurch gekennzeichnet, daß** auf den Flächen der Referenzstruktur 26 mit der Spitze eines Zeigers 24 in Kontakt mit der Referenzstruktur 26 beliebige, vorzugsweise die jeweilige Fläche ausnutzende Bahnkurvenabschnitte 34 bis 34"" abgefahren werden und daß während dieses Abfahrens die Koordinaten der Spitze des Zeigers 24 mittels des Lageerfassungssystems 18 in dessen Koordinatensystem punktweise erfaßt werden.

5. Verfahren nach einem der Ansprüche 1-3,
**dadurch gekennzeichnet, daß** aus den ermittelten Koordinaten der Antastpunkte im Koordinatensystem des Lageerfassungssystems 18 und aus den bekannten Koordinaten der Antastpunkte im Koordinatensystem der bildgebenden Diagnostikeinrichtung rekursiv eine Transformationsmatrix zwischen den beiden Koordinatensystemen errechnet wird.

6. Verfahren nach Anspruch 4,
**dadurch gekennzeichnet, daß** mit Hilfe eines iterativen Rechenverfahrens die Referenzstruktur 26 virtuell im Koordinatensystem des Lageerfassungssystems 18 verschoben und verschwenkt wird und daß für jede Orientierung der Referenzstruktur 26 die Summe der Quadrate der Abstände der Antastpunkte zu der bekannten Oberfläche der Referenzstruktur 26 ermittelt wird und diejenige Orientierung der Referenzstruktur 26 zur Berechnung der Transformationsmatrix herangezogen wird, bei der die Summe der Abstandsquadrate ein Minimum einnimmt.

7. Verfahren nach einem der Ansprüche 1-6,
**dadurch gekennzeichnet, daß** die Ermittlung der Koordinaten der Spitze des Zeigers 14, 24 mittels des Lageerfassungssystems 18 nur dann erfolgt, wenn die Zeigerspitze mit einer Anpreßkraft an die Referenzstruktur 16, 26 gedrückt wird, die eine vorgegebene obere und eine untere Grenze aufweist, so daß sicher gestellt ist, daß die Spitze des Zeigers 14, 24 einen Kontakt zu der Referenzstruktur hat und diese nicht durch den Antastvorgang verlagert.

8. Verfahren nach einem der Ansprüche 1-6,
**dadurch gekennzeichnet, daß** die Ermittlung der Koordinaten der Spitze des Zeigers 14, 24 mittels des Lageerfassungssystems 18 in einem Zeitfenster unmittelbar nach dem Zeitpunkt ermittelt wird, zu dem ein elektrischer Kontakt zwischen der Spitze des Zeigers 14, 24 und der Referenzstruktur 16, 26 nachgewiesen wurde.

9. Verfahren nach einem der Ansprüche 1-6,
**dadurch gekennzeichnet, daß** die Ermittlung der Koordinaten der Spitze des Zeigers 14 mittels des Lageerfassungssystems 18 bei Vorhandensein einer Sterilabdeckfolie zwischen der Spitze des Zeigers 14 und der Referenzstruktur 16 nach visueller Kontrolle der Faltenfreiheit der Sterilabdeckfolie im Bereich des Antastpunktes erfolgt und bei der Ermittlung der Transformationsmatrix die Dicke der Folie berücksichtigt wird.

10. Verfahren nach einem der Ansprüche 1-9,
**dadurch gekennzeichnet, daß** die bildgebende Diagnostikeinrichtung während des Abtastens der Referenzstruktur 16, 26 eine gegenüber einer mechanischen Krafteinwirkung weitgehend unempfindliche Orientierung einnimmt und die Verstellachsen der bildgebenden Diagnostikeinrichtung arretiert sind.

11. Verfahren nach einem der Ansprüche 1-2, 4-10,
**dadurch gekennzeichnet, daß** von einer Bedienperson der Zeiger 14, 24 beim Antasten eines Antastpunktes derart im Raum orientiert wird, daß vorzugsweise alle Marken 15, 25 des Zeigers 14, 24 von dem Lageerfassungssystem 18 erfaßt werden.

12. Verfahren nach einem der Ansprüche 1-11,
**dadurch gekennzeichnet, daß** die Koordinatentransformation so lange ihre Gültigkeit behält, bis die Steuerung der bildgebenden Diagnostikeinrichtung detektiert, daß eine über den Auslegungsverstellbereich hinaus gehende Verstellung der bildgebenden Diagnostikeinrichtung stattgefunden hat.

13. Vorrichtung zur Durchführung des Verfahrens nach einem der Ansprüche 1-12,
**dadurch gekennzeichnet, daß** die Referenzstruktur 16 wenigstens drei Antastpunkte aufweist, die von einem Zeiger 14 mit von einem Lageerfassungssystem 18 erfaßbaren Marken 15 antastbar sind und von denen drei Punkte ein ungleichschenkliges Dreieck bilden und daß die Antastpunkte an der Bildaufnahmeeinheit der bildgebenden Diagnostikeinrichtung angeordnet sind.

14. Vorrichtung zur Durchführung des Verfahrens nach einem der Ansprüche 1-12,
**dadurch gekennzeichnet, daß** die Referenzstruktur 26 wenigstens drei Antastpunkte aufweist, die von einem Zeiger 24 mit von einem Lageerfassungssystem 18 erfaßbaren Marken 25 antastbar sind und von denen drei Punkte ein ungleichschenkliges Dreieck bilden und daß die Antastpunkte an einer Tragstruktur der bildgebenden Diagnostikeinrichtung angeordnet sind.

15. Vorrichtung nach Anspruch 14,
**dadurch gekennzeichnet, daß** die bildgebende Diagnostikeinrichtung wahlweise ein Computertomograph, eine Röntgendiagnostikeinrichtung, ein Kernspinresonanztomograph oder eine bildgebende Ultraschalleinrichtung ist.

16. Vorrichtung nach Anspruch 14,
**dadurch gekennzeichnet, daß** die Tragstruktur eine Horizontalführung 3 und die bildgebende Diagnostikeinrichtung eine mobile und wahlweise für 2D- oder 3D-Navigation ausgerüstete Röntgendiagnostikeinrichtung ist.

17. Vorrichtung zur Durchführung des Verfahrens nach einem der Ansprüche 1-12,
**dadurch gekennzeichnet, daß** die Referenzstruktur 16, 26 aus einem Körper mit einer bekannten, anisotropen Oberflächengeometrie besteht, dessen Oberfläche mittels eines Zeigers 14, 24 antastbar ist. wenigstens drei Antastpunkte aufweist, von denen drei Punkte ein ungleichschenkliges Dreieck bilden und daß die Antastpunkte an der Bildaufnahmeeinheit der bildgebenden Diagnostikeinrichtung angeordnet sind.

18. Vorrichtung zur Durchführung des Verfahrens nach Anspruch 3,
**dadurch gekennzeichnet, daß** die Referenzstruktur 26 an den Orten der Antastpunkte anisotrope Vertiefungen aufweist, die in einer eindeutigen Orientierung passgenau und lösbar die Spitze des Zeigers 24 aufnehmen

19. Vorrichtung nach Anspruch 18,
**dadurch gekennzeichnet, daß** die Referenzstruktur 26 an den Orten der Antastpunkte pyramidenstumpfförmige Vertiefungen 32 aufweist, die in einer eindeutigen Orientierung passgenau und lösbar die Spitze des Zeigers 24 aufnehmen

20. Vorrichtung nach Anspruch 18,
**dadurch gekennzeichnet, daß** die anisotrope Spitze des Zeigers 24 in den anisotropen Vertiefungen mittels Haltevorrichtungen während des Zeitraumes der Lagebestimmung des Zeigers 24 lösbar gehalten ist.

21. Vorrichtung zur Durchführung des Verfahrens nach einem der Ansprüche 1-12,
**dadurch gekennzeichnet, daß** die Referenzstruktur 16, 26 und die Spitze des Zeigers 14, 24 elektrisch leitfähige Oberflächen aufweisen und daß zwischen der Referenzstruktur 16, 26 und der Spitze des Zeigers 14, 24 Vorrichtung zur Messung des Übergangswiderstandes geschaltet ist, die den Zeitpunkt der Berührung der Spitze des Zeigers 14, 24 an der Referenzstruktur 16, 26 ermittelt und das Lageerfassungssystem 18 veranlaßt, die Bestimmung der Koordinaten der Spitze des Zeigers 14, 24 vorzunehmen.

22. Vorrichtung zur Durchführung des Verfahrens nach einem der Ansprüche 1-12,
**dadurch gekennzeichnet, daß** die Spitze des Zeigers 14, 24 einen an eine Auswerteelektronik angeschlossenen Kraftmeßsensor aufweist, wobei die Auswerteelektronik das Lageerfassungssystem 18 veranlaßt, die Bestimmung der Koordinaten der Spitze des Zeigers 14, 24 vorzunehmen, wenn die Antastkraft eine die Berührung sicher stellende Minimalkraft überschritten und eine die Verlagerung der Referenzstruktur 16, 26 gefährdende Maximalkraft unterschritten bleibt.

23. Vorrichtung zur Durchführung des Verfahrens nach einem der Ansprüche 1-12,
**dadurch gekennzeichnet, daß** die Spitze des Zeigers 14, 24 eine berührungslos arbeitende Meßeinrichtung zur Bestimmung der Entfernung der Spitze des Zeigers 14, 24 von der Oberfläche der Referenzstruktur in Richtung der Achse des Zeigers 14, 24 aufweist und daß die Meßeinrichtung im Zeitpunkt der Ermittlung der Koordinaten und der Richtung der Spitze den ermittelten Abstand an das Lageerfassungssystem übermittelt.

24. Vorrichtung zur Durchführung des Verfahrens nach einem der Ansprüche 1-12,
**dadurch gekennzeichnet, daß** die Referenzstruktur 16, 26 bei bereits im Einsatz befindlichen bildgebenden Diagnostikeinrichtungen nachrüstbar ist.

25. Vorrichtung zur Durchführung des Verfahrens nach einem der Ansprüche 1-12,
**dadurch gekennzeichnet, daß** Lageerfassungssystem 18 wahlweise ein optisches Infrarotmeßsystem, ein optisches Lasermeßsystem, ein Magnetfeldmeßsystem oder ein Meßsystem zur Messung eines elektrischen Feldes aufweist.

26. Vorrichtung zur Durchführung des Verfahrens nach einem der Ansprüche 1-12,
**dadurch gekennzeichnet, daß** bei Vorhandensein von mehreren Referenzstrukturen 16, 26 an der bildgebenden Diagnostikeinrichtung diese jeweils unterschiedliche charakteristische Oberflächengeometrien oder Anordnungen von Antastpunkten aufweisen, wodurch eine automatische Zuordnung der von dem Lageerfassungssystem 18 ermittelten Koordinaten der Antastpunkte zu den jeweiligen Referenzstrukturen 16, 24 sichergestellt ist.
